# EUROPEAN PATENT SPECIFICATION

(11) **EP 2 592 993 B1**
(45) Date of publication and mention of the grant of the patent: **10.10.2018**
(21) Application number: 11758542.2
(22) Date of filing: 15.07.2011
(51) Int. Cl.: A61B 1/24, A61B 5/00

(54) **DETECTING DEVICE**
DETEKTOR
DISPOSITIF DE DÉTECTION

(30) Priority: 15.07.2010 GB 201011913
(43) Date of publication of application: 22.05.2013
(73) Proprietor: Calcivis Limited, Midlothian EH16 4UX (GB)
(72) Inventor: PERFECT, Emma, Edinburgh EH12 5NP (GB); LONGBOTTOM, Chris, Fife DD6 8DW (GB); HAUGHEY, Anne-Marie, Glasgow G31 3EW (GB)
(74) Representative: Jacob, Reuben Ellis
(86) International application number: PCT/GB2011/051339
(87) International publication number: WO 2012/007769

(56) References cited:
- EP-A2- 1 252 859
- WO-A1-2008/113493
- US-A1- 2005 003 323
- US-A1- 2008 082 000
- US-A1- 2008 160 477
- US-A1- 2010 034 750

## Description

The invention relates to a detecting device suitable for detecting low levels of luminescence and/or fluorescence.

Detecting low levels of luminescence and/or fluorescence is useful in a number of applications, in particular where an assay uses luminescent or fluorescent emissions to indicate the presence of a particular disease or condition. The light detected using such assays may be used for skin and/or intraoral imaging. However, luminescent and fluorescent signals tend to be weak and therefore can be difficult to detect. This is particularly difficult in the presence of ambient light which can overwhelm the signal.

One example of an assay employing luminescent emissions to indicate that certain symptoms are present has recently been developed. This method uses photoproteins or other light-emitting substances for the detection of demineralising tooth surfaces. The photoproteins emit a luminescent signal in the presence of the tree ions released during demineralisation, and this information can be used as a biomarker of tooth decay (caries) or erosion. Other luminescent or fluorescent markers which are responsive in this way to other biomarkers of disease can also be used for examining the presence of plaque, skin or genito-urinary conditions for which there is an associated biomarker. The light signal generated in these assays is relatively low, and a large amount of ambient light is present in the associated locations of the mouth and skin.

Document US2010/034750 discloses a composition comprising photoproteins for the detection of tooth demineralisation. Devices have been designed for the purpose of detecting light in the enclosed or confined (but nevertheless exposed to ambient light) spaces necessary for the assessment of intra-oral, genito-urinary or skin conditions. These prior art devices tend to be relatively insensitive to low levels of luminescence, particularly in the presence of ambient light. In addition, further problems are encountered where it is necessary to produce an image from the detected light; the prior art devices tend to have a pen-like shape, which is held in the cavity by the operator. Being mobile and hand-held, the devices tend to be insufficiently stable to produce a clear image over the extended exposure times required to detect the low levels of light. Documents US2005/003323, US2008/082000 and US 2008/160477 disclose examples of a intraoral detection apparatus.

It is an object of the invention to provide a detection device for detecting low levels of luminescent or fluorescent emissions. This includes those regions or surfaces that are exposed to ambient light.

The invention is defined by the independent claims.

In a first aspect of the invention, there is provided a detection device comprising a proximal end and a light-transmitting portion, wherein the proximal end comprises a light detection means and the light-transmitting portion comprises means for transmitting light along a path from a surface of interest to the light detection means, characterised in that the light transmitting portion is optimised for use in detecting a low light signal generated by fluorescence or luminescence in conditions of ambient light; and wherein the device is adapted to detect the presence of a disclosing substance which has been applied to a surface of interest. This combination of features allows low levels of luminescence and/or fluorescence to be detected whilst shielding the detector from ambient light. In other words, the means for reducing ambient light prevents the signal from the surface of interest, which may be indicative of a disease state, from being mixed with ambient light, creating a false positive result and ensuring that any signal generated is not overwhelmed by the ambient light.
Preferably, the light-transmitting portion is positioned adjacent to and is adapted to fit around the surface of interest. Advantageously, the light-transmitting portion will be held securely in place while light emitted therefrom is being detected. This may be of assistance in situations wherein an operator wishes to produce an image from the detected light.
In the context of the present invention, the proximal end refers to the end of the device proximal to the user of the detection device, in contrast to the end of the device that is proximal to a patient when the device is in use on that patient. In one embodiment, the device may be hand-held, such that the proximal end of the device corresponds to the end that is gripped by an operator during use. Advantageously, the proximal end may comprise a handle or other gripping means.
In one embodiment, the whole of the light-transmitting portion is positioned adjacent to and adapted to fit around the surface of interest. However, in an alternative embodiment, only part of the light-transmitting portion is positioned adjacent to and adapted to fit around the surface of interest. Conveniently, only the part of the light-transmitting portion that is optimised for use in detecting a low light signal generated by fluorescence or luminescence in conditions of ambient light is positioned adjacent to and adapted to fit around the surface of interest.

Preferably, the substance applied to the surface of interest may comprise an optical marker. Advantageously, an operator will therefore be able to detect the luminescent and/or fluorescent signal as soon as the disclosing substance has been applied to the surface of interest. Typically, the surface of interest is the surface of a tooth.

Preferably, the low light signal is detected in close succession to the application of the disclosing substance to the surface of interest. Thus, the operator can detect the signal without any loss of luminescence and/or fluorescence intensity in the time between applying the disclosing substance and detecting the signal, time which would be lost if it was necessary to use separate instruments for the application and the detection steps. This is important if the signal is generated quickly and transiently.

Typically, the device comprises an elongate member. Preferably, the elongate member assists with insertion of the device into the mouth of a patient.

Preferably, the proximal end of the device further comprises a lens. Preferably, the lens is located at a position along the path for transmitting light from a surface of interest to the light detection means. This feature will allow the operator to not only detect the total luminescence and/or fluorescence emitted, but also to produce a resolved image of the surface. In one embodiment, a second lens may be positioned downstream of the first lens to magnify the image that reaches the detector.

Preferably, the means for transmitting light is a prism or a reflective surface. In one embodiment, the device comprises a mirror. The mirror would be useful where the device is to be used for intra-oral imaging because the dental practitioner would be able to locate the correct area and position the imaging device correctly in the mouth before applying the disclosing substance. The mirror could also be used to check that the disclosing substance had been applied correctly to the surface of interest. However, the skilled person would understand that any other optical component designed for transmitting, reflecting or refracting light may be used in accordance with the present invention.

Preferably, the prism or reflective surface is coated with a bandpass, polarising or dichroic filter material. The provision of these filters can remove unwanted wavelengths and/or polarities of light so that the signal to noise ratio will be improved. Preferably, the prism or reflective surface is coated with a material that will reflect, absorb, transmit or refract certain wavelengths of light. In those embodiments wherein the wavelength of the signal is known, the material will allow a signal having a wavelength of interest, or the luminescent and/or fluorescent signal, to be transmitted without the presence of background noise.

Typically, the prism or reflective surface is positioned upstream of the light detection means. Advantageously, the positioning of the prism or reflective surface will assist with the transmission, reflection or refraction of light. By 'upstream' it is meant that the prism or reflective surface is positioned at a location along the path of light between the light transmitting means and the light detection means.

Preferably, a light-transmitting material is connected to the light-transmitting portion and acts as a 'light pipe'. This acts to reduce the size of the air layer between the surface and device which helps with focusing and production of a resolved image, and comprises gel, glass, fibre optics or other crystalline material that transfers light effectively.

Preferably, the light-transmitting portion is arranged at an angle permitting light from a specific area of the surface of interest to be received. For example, in the context of intra-oral imaging, the light-transmitting portion may be arranged such that light can be transmitted from one, two, three or more teeth or even a whole lower or upper arch of teeth, so that an analysis can be performed on a larger area whilst still using a device with a small diameter that can fit into small spaces such as the mouth.

Preferably, the device further comprises a skirt surrounding an end of the light-transmitting portion. Preferably, the skirt is provided to assist with positioning the light-transmitting portion adjacent to the surface of interest. Typically, the skirt comprises a thin seal provided within a compartment for retaining a disclosing substance and wherein compression of the skirt against the surface of interest breaks the seal and releases the disclosing substance therefrom. Advantageously, the skirt acts as a shield to reduce interference from ambient light during detection of the signal.

In one embodiment, the skirt may comprise a flexible material so that it can be 'moulded' to the individual patient's oral surfaces. Alternatively, the skirt may be manufactured for an individual and therefore pre-moulded and set in the correct shape. In the latter case, the skirt would be of a fixed shape, once moulded.

Luminescence and fluorescent light imaging is difficult to perform, because the luminescent and/or fluorescent signal may be relatively weak. This is particularly true for intraoral or skin imaging. Preferably, the light detection means is sufficiently sensitive to detect light emitted at a rate of 9.63 x 10⁶ photons s⁻¹. Preferably, the light detection means is selected from the group consisting of a luminometer, fluorometer, spectrophotometer, camera, charge coupled device (CCD), complementary metal-oxide semiconductor (CMOS), charged injection device (CID), avalanche photodiode (APD), single photon detector, digital camera, intensified camera, photographic film, photometric detector, photomultiplier tubes (PMT), microelectromechanical system (MEMS), a human eye or a combination of these. Typically, the APD may include Geiger mode of single photon avalanche photodiodes (SPADS),

Preferably, the light-transmitting portion comprises at least one light source selected from the group consisting of an LED, laser, lamp or a hole cut into the device to allow ambient light to enter therein, thereby allowing transmittance of light onto the surface to be assessed. Additionally, the light-transmitting portion may include an optical fibre or a glass rod connected to one of these light sources.

Preferably, the light source is an LED. This light source allows the user to illuminate and take an image of the area prior to the detection and analysis of luminescent and/or fluorescent light Preferably, the LED has an "off" setting to allow the user to turn off the illumination whilst producing the luminescence and/or fluorescence image. The luminescence and/or fluorescence image can then be compared with the illuminated image, in order to determine exactly which areas are emitting light. This could be particularly useful, for example, where the luminescence and/or fluorescence is being emitted by a photoprotein that has come into contact with free ions that are released by a tooth surface due to demineralisation. This would allow the user to determine the location of the demineralisation on the tooth.

Preferably, the proximal end of the device is reusable and the light-transmitting portion is disposable. The light-transmitting portion is likely to come into close contact with body tissues, such as intraoral or skin surfaces. As a result, infection control issues will be raised. It can be particularly cumbersome, particularly in a busy medical or dental practice, to continually disinfect a device between uses. It would be much easier, quicker and convenient to discard and replace the light-transmitting portion.

Preferably, the whole device or part of the device is autoclavable. Although the proximal end is unlikely to come into contact with body tissues, it is desirable for a medical device to be suited to sterilisation, chemical or otherwise. In addition, the device may be disposable. These properties of the device reduce the risk of contamination and possibility of cross-infection of a patient using the device.

Preferably, the device is operably attached to a computer. Typically, the device is connected to a computer via wireless means. The advantages of this are numerous. The user would be able, using software programs installed on the computer, to produce images, manipulate or analyse the data, and save data produced by the device. Preferably, the device is connected to the computer via wireless means. This would ensure that the user would not be concerned with wires trailing around the lab or surgery, which could be inconvenient or a trip hazard. Furthermore, it can be difficult to produce a clear image of a weak luminescence and/or fluorescence signal, especially if a camera is moving. If wires were attached to the device, this could cause the device to be 'dragged' due to the weight of the wires, preventing the camera from remaining still.

Preferably, the light-transmitting portion farther comprises means for stabilising the device in an intraoral cavity of a patient. Preferably, the means for stabilising the device comprises a bite block positioned to extend from the device towards and through a patient's teeth. Advantageously, the means for stabilising the device may be used to hold the detector in place, so that a stable image may be produced. The means for stabilising the device could also be provided as a clamp adapted to "fit" over a tooth.

Preferably, a disclosing substance may be applied to a surface of interest using an injector device or gun. In one embodiment, the gun is a spray gun. The substance would therefore be conveniently injected directly onto the surface to be imaged, such that the image may be taken as soon as the luminescent and/or fluorescent signal is emitted. This is advantageous in situations wherein the disclosing substance emits a signal very quickly after application, as it avoids the need to remove the injector device after injection and subsequently position the detector device correctly in time to 'catch' the signal. Preferably, the injector or gun comprises a nozzle that allows the disclosing formulation to be sprayed in a fine spray onto the surface of interest.

In another embodiment, the device further comprises a camera.

In accordance with a second aspect, the present invention provides a kit comprising a detection device in accordance with the first aspect of the invention and further comprising a mouth shield.

In one embodiment, the mouth shield may form part of the device. Alternatively, the mouth shield may be supplied in a kit according to the second aspect. The mouth shield could be placed in or around the mouth and the device, in order to Further exclude any ambient light from entering the area to be imaged.

In accordance with a third aspect, the present invention provides a method of detecting a low light signal generated by fluorescence or luminescence in conditions of ambient light, using a detection device in accordance with the first aspect, the method comprising the steps of:
a. placing the light-transmitting portion of the device adjacent to a surface of interest;
b. applying a disclosing substance to the surface of interest; and
c. detecting the light emitted from the surface of interest.

Preferably, the disclosing substance is released from the detection device and applied to the surface of interest. By applying the disclosing substance with the same device as that used to detect the light emitted from the surface, the method is less complicated than methods that use separate applicator and detector devices. In particular, this is useful if the disclosing substance acts very quickly and transiently, in which case there would be insufficient time to position a separate detection device correctly after application of the disclosing substance with a separate application device. The application may be by injection, or spraying, and the disclosing substance may be a powder, a liquid or a gel.

Preferably, the disclosing substance is retained within a compartment of the skirt by a thin seal and released onto the surface when the skirt is pressed onto the surface of interest resulting in the seal being broken. This provides a convenient method for applying the disclosing substance, which does not require any additional injector device. This would reduce the complexity and cost of manufacture, and also the weight and bulk of the device.

Preferably, the method further comprises the step of producing an image of the surface of interest by focusing the light emitted therefrom through a lens provided on the device. This would assist a medical or dental practitioner in determining the extent of the luminescence and/or fluorescence and the precise area from which the light is emitted.

Preferably, the method further comprises the step of producing an image of the surface of interest prior to application of the disclosing substance. This would allow an operator to be able to overlay the image of the luminescence and/or fluorescence with the normal, pretreatment, ambient or artificial light image, in order to precisely locate where the luminescence and/or fluorescence is being emitted from and therefore where an area of caries may be developing.

In one embodiment, the disclosing substance comprises a powder that dissolves in the presence of saliva. This would be of particular use where the surface to be imaged is an intraoral surface. Another advantage of this feature is that the disclosing substance is active in solution, and therefore the powder could be stored in a stable inactive form for long periods of time.

Alternatively, the disclosing substance may be a gel and may fill part of, or the entire space between a tooth and the means for transmitting light. This would help ensure a resolved, focused image is obtained and increase the efficiency of light transfer, so improving the sensitivity of the device. Typically, the viscosity of the disclosing substance can affect the speed of detection and the magnitude of the signal. An increase in the viscosity of a gel correlates with a decrease in signal strength. Therefore, the greater the viscosity of the gel the greater the detection time required to obtain a useful image. However, different viscosity gels may be advantageous on different parts of the oral cavity. For example, more viscose gels may be advantageous for use on certain surfaces, e.g. the smooth surfaces on some of the premolars. On these surfaces, a more viscose gel may be required to slow the movement of gel down the surface and so enable a localized signal to be captured. Less viscous gels may provide a better option for occlusal surfaces of permanent molars as the fissures help keep this pooled, also if the gel is too viscose it may not get into the bottom of the fissures to provide a complete picture of the surface. Gels of different viscosity could be used independently or in combination to produce a complete picture of a surface, a tooth or the entire oral cavity.

Preferably, the disclosing substance is applied to the surface of interest through the application of pressure thereto. The disclosing substance may be contained within a compartment provided within the skirt and released when the skirt is pressed onto the surface of interest. A thin seal may act as a barrier to prevent the disclosing formulation being applied to surface of interest until pressure is applied, e.g. by pressing the skirt onto the surface.

Preferably, a reference point may be applied to the surface of interest prior to application of the disclosing substance. The reference point may be used to align images that have been taken at different times. It may also be used to align images taken using different equipment, or to align the equipment itself. The reference point could be used to standardise light output between images. The reference point may be a spot of ion-containing material, or other distinctive mark. A ruler may be etched onto the device, for example onto a fibreoptic plate for measurement and calibration purposes.

The device will now be described by way of example and with reference to the following figures, in which;
Figure 1 shows an embodiment of the light detection, proximal and imaging portions of the device, where the device is square or cuboidal in cross section;
Figure 2 shows an embodiment of the light detection, proximal and imaging portions of the device, where the device is circular in cross section;
Figure 3 shows an embodiment of the device in situ;
Figure 4 shows the intensity of light emitted from a freshly extracted tooth with an active caries lesion;
Figure 5 shows the intensity of light emitted from a G2 and a G3 Glowelt®;
Figure 6 is a graph comparing the average light intensities of a luminescent area form the freshly extracted tooth and the G2 and G3 Glowell® units;
Figure 7 shows a luminescence image of Glowell® units obtained in darkness;
Figure 8 shows a luminescence image of Glowell® units obtained in ambient light using a skirt;
Figures 9 and 10 show the placement of the filter;
Figure 11 shows a luminescence image of Glowell® units obtained in ambient light using a filter;
Figure 12 shows a luminescence image of Glowell® units obtained in ambient light using a filter and a skirt in combination;
Figures 13a and 13b shows a photo of a luminescence detection device and a schematic of the same device (comprising a prism, a lens and a CCD), which device has been used to capture images from occlusal and free smooth surfaces of teeth;
Figure 14a shows a luminescence image of Glowell® units obtained in ambient light using a skirted embodiment of the device, as shown in Figure 14b; and
Figure 15 shows luminescence images of two freshly extracted teeth captured with a skirted embodiment of the device, as shown in Figure 14b.
Figure 16 shows the influence on gel viscosity on the speed and magnitude of the light signal generated.
Figure 17 shows the feasibility of combining images collected over short period of time to generate a higher signal and so clearer image.

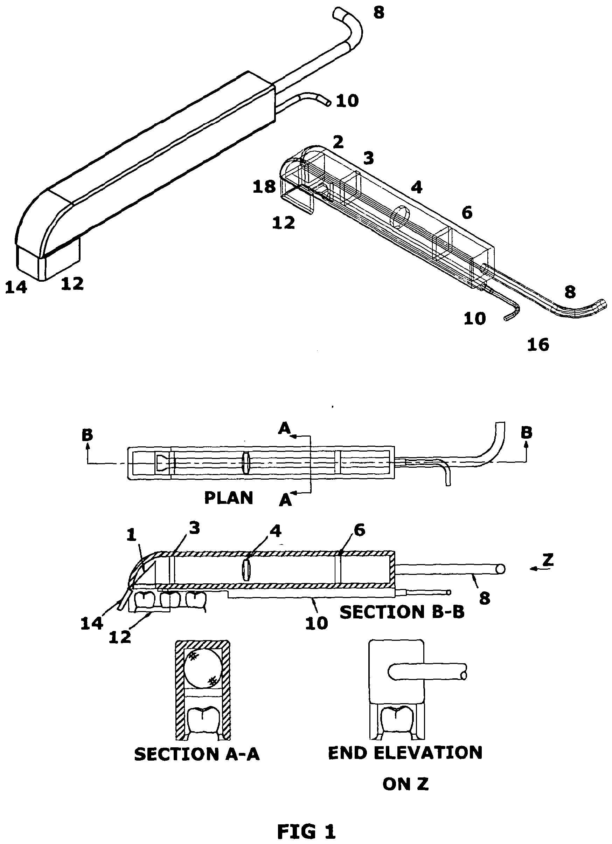

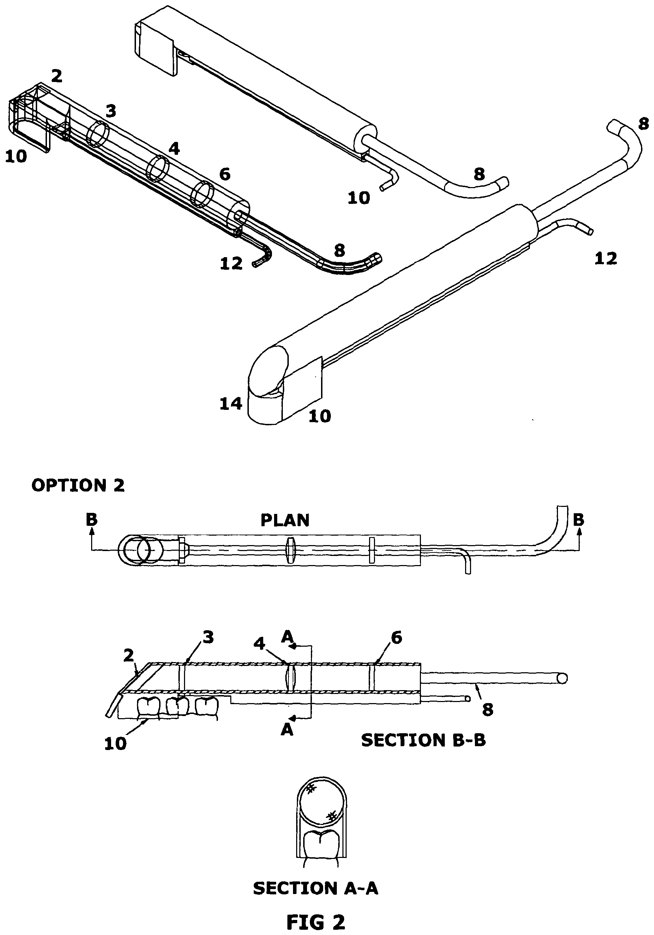

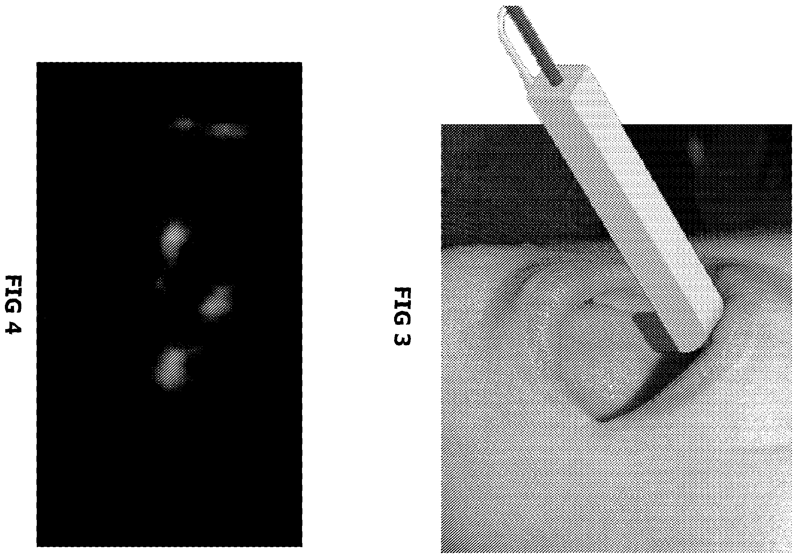

One embodiment of the detection device according to the invention is shown in Figures 1 and 2 and comprises a prism, mirror or reflector 2, a lens or filter 3, a detector 6, a wire or wireless emitter 8, a skirt 10, an injector or spray gun 12 and a movable skirt 14. The device is elongated and rectangular or cylindrical in shape with a proximal end or handle 16 and a distal or light-transmitting portion 18. The proximal end 16 and light-transmitting portion 18 are preferably aligned along one plane so that the device is suitable for fitting into a patient's mouth, and may comprise either two separate members joined together, or a single member as illustrated in Figure 1. In situations wherein the device is used for intra-oral imaging, the light-transmitting portion 18 is inserted into a patient's mouth, while the proximal end 16 is generally located outside the patient's mouth and held by an operator, as shown in Figure 3. The luminescent signal is reflected or refracted from a prism, mirror or reflector 2, or a dichroic filter located in the light-transmitting portion 18 so that it can be reflected towards the detector 6, wherein the detector 6 is located at the proximal end 16. The light-transmitting portion 18 may be disposable, to prevent the transmission of infections between patients. In accordance with one embodiment of the present invention, when using a device comprising only the lens 3, total light can be detected from the surface of interest. Through addition of lens 4 as illustrated in Figure 1, it would be possible to create an image of the regions on the surface emitting light on the detector 6. The lens 3, 4 may be a biconvex, planoconvex or positive meniscus lens.

The light transmitted from the surface of interest may enter through a hole provided on the light-transmitting end of the device, the hole preferably being square or rectangular. This light may either enter a prism 2 and be refracted such that it may travel towards a detector 6, or the light may be reflected from a reflective surface such as a mirror 2. The prism, mirror or reflector 2 may be coated with a bandpass material, a dichroic material, or another suitable material, in order to alter the wavelengths transmitted. In order to improve resolution, the light may travel through a collimator from the prism, mirror or reflector 2 towards the detector 6. Alternatively, a glass rod, or rods or lenses, or fibreoptic bundle may be provided, through which light may travel from the prism or reflective surface 2 and towards the lens or detector 6 (Figure 1).

As discussed previously, the device according to the present invention comprises features that are designed to reduce the ambient light. In one embodiment, the body of the light-transmitting portion 18 and the proximal end 16 comprise an opaque material. A skirt 10 may be provided to prevent ambient light from entering into the light-transmitting portion 18 or from reaching the detector 6. The skirt 10 preferably comprises at least one light absorbing material. In one embodiment, bandpass filters or dichroic filters may be used, which allow only the desired wavelengths of light to reach the detector 6. Finally, a detector 6 may be used that is sensitive only to specific wavelengths of light, such as those emitted from the surface of interest. In another embodiment, the light-transmitting portion 18 is disposable. Preferably, the light-transmitting portion 18 comprises a type of plastics material that is deformable upon addition of a disinfectant solution. In situations wherein the plastics material deforms upon exposure to a disinfectant solution, the light-transmitting portion cannot be reused. As a result, the operator will need to buy a new light-transmitting portion for treatment or examination of each individual patient. Thus, profitability of the device will be improved for the manufacturer.

In one embodiment, a band pass filter 3 may be used (see Figure 1) to block out any wavelengths of light that are not attributable to the luminescence signal of interest. When using aequorin or obelin for detection of free ions on an intraoral surface, bandpass filters with narrow bands centred around 465 or 485 nm, respectively, may be used for the detection of the luminescent signal. These filters may be positioned upstream of the detection device, along the path of light transmission, so that light is filtered before reaching the detector 6 located at the proximal end of the detection device.

The detector 6 may provide information regarding the amount of light collected so as to provide information on the intensity, spectra, speed of signal generation, or may generate an image illustrating spatially where the light is generated. In one embodiment, a CCD detector may be used. Such detectors have the advantage of being sensitive to particular wavelengths of light. In the embodiment wherein a luminescent signal is detected from either aqueorin or obelin, a CCD which is sensitive to blue light in a narrow region, typically around 465 or 485 nm, respectively, may be used to detect light in a luminescent assay, and a red or green sensitive CCD could be used to obtain a visible image of the tooth surface.

Intraoral cameras have seen high levels of adoption by dentists over the last decade. These cameras can produce clearly defined images that show details which may be missed by standard mirror examinations, as well as providing an excellent educational and communication tool, since they allow the patient to see inside their own mouth during an examination. The cameras often incorporate a CCD and lends but can only take images in bright working conditions (usually achieved with LEDS). Despite a number being tested for use with disclosing material, they have not proved suitable for imaging the low amounts of light produced by the luminescent marker used here.

The device of the present invention may be used in: detecting the presence of caries lesions, including active caries lesions, after application of an ion-sensitive luminescence marker or a fluorescence marker; determining erosion susceptibility of a tooth following acid challenge and application of an ion-sensitive luminescence marker or a fluorescence marker; determining the site of root-dentine hypersensitivity following a specific food/liquid challenge and the application of an ion-sensitive luminescence marker or a fluorescence marker; determining localisation of remineralisation product, sealant and infiltrants following acid challenge of a tooth of a patient and application of an ion sensitive luminescence marker or a fluorescence marker; or determining the localisation or investigating area of interest of pre-cancerous or cancerous areas in oral mucosa, genitourinary tract, or skin following the addition of a biomarker of disease tagged with an optical marker.

In some embodiments of the invention, a skirt 10 is provided and positioned around the device. Advantageously, the skirt 10 may secure the device in position to avoid image blurring and increase the comfort of the user. The skirt 10 may also help protect the device from saliva or other fluids which may interfere with a signal generated from a marker. The skirt 10 may be rigid, or flexible to enable the skirt 10 to mould to the surface and prevent ambient light from entering the device. Preferably, the skirt 10 comprises a black rubber material. The skirt 10 may be used to assist in the stabilising of the light-transmitting portion 18 of the device within the mouth of a patient. In one embodiment, the skirt comprises individual pieces which may be removed, such that it is possible to use the device to view the buccal, lingual, palatial and labial surfaces of the teeth and mucosal surfaces if desired. Therefore, all intraoral surfaces, with the exception of approximal surfaces, may be imaged using the device of the present invention.

The walls of all parts of the device preferably comprise a rigid material having a black colour, to assist in reducing as much of the ambient light as possible. The walls of the device may comprise a number of materials, such as glass, plastic, paper, cardboard or metal. Polystyrene, polyvinylchloride (PVC), polyacrylic or polypropylene are particularly suitable for this purpose. If the material is polypropylene, the device could be autoclavable. Alternatively, a disposable and sterile sheath may be placed over the device to protect the device and patients from contamination, or the device may be sterilised using ethanol or chemical disinfectant before use.

A mouth shield may be used with or added to the device in order to improve the detection of low light signal in conditions of ambient light. This shield may be opaque to block light of all wavelengths, or coloured to block out light having a particular wavelength, in order to reduce background noise and improve quality of the signal. In one embodiment, the shield may block light of those wavelengths that correspond to those expected to be emitted from the surface of interest, so that any signal detected can be attributed to a signal of interest rather than background light. A coloured mouth shield may allow a dental practitioner or doctor to see objects within the mouth, but prevent light of interfering wavelengths from entering the mouth.

The prism or reflective surface 2 may be mounted on a flexible mount or an angled mount, such that it may be adjusted to detect a luminescent and/or fluorescent signal from different sized areas if necessary. For example, where the device is used intra-orally, the transmitting or reflecting means may be angled to enable light from a larger area (e.g. three occlusal surfaces) to be collected in one step and reduced in size through appropriate positioning. Positioning the prism or reflector 2 at a greater angle from the plane of the main member of the device has the advantage that light from a smaller area would be detectable. If the prism or reflector 2 were positioned at a smaller angle from the plane of the main member of the device, a larger area may be detectable.

The use of an angled device may have cost benefits, because it is possible that a CCD with a smaller surface area could be used. In the embodiments wherein the device is used for intraoral imaging, by positioning the transmitting or reflecting means at a specific angle, which angle may be adjusted, the time taken for a dental assessment could be reduced as more surfaces can be assessed simultaneously without needing to move the device. This is demonstrated in Figure 2 which comprises a prism, mirror or reflector 2, a lens or filter 3, a detector 6, a wire or wireless emitter 8, a skirt 1 0, an injector or spray gun 12 and a moveable skirt 14. The skilled person would understand that, when used intraorally, the device will be adaptable for investigation of the following: both arches simultaneously; upper and lower arch separately; quadrants of the arch separately; single teeth; and the soft tissues e.g. the gingivae, the tongue, the oral mucosa and the pharynx.

The device may, in some embodiments, be capable of both transmitting light and detecting light. The device may comprise an excitation light source if it is required for use in fluorescence assays. In order to allow images of the region of interest to be produced, the device may further comprise a light source. In embodiments wherein the device comprises optical fibre bundles, some fibres will transmit light and some will receive the emitted signal. Alternatively a light source, such as an LED, a laser or a lamp, may be placed on the device at a position adjacent to the surface being imaged. In the embodiments wherein tooth surfaces are to be imaged, the light may be transmitted through the teeth of a patient, so as allow imaging. The light source may be provided as a ring of LEDs around the opening such that the device may be used to obtain good quality, clear images of the teeth. Alternatively, the light source may be provided as a ring of LEDs around the lens. The LEDs may have an "off" setting so that only the luminescence and/or fluorescence signal is detectable when desired. In accordance with the present invention, a method is also provided for controlling the entrance of light into the device.

In accordance with the present invention, the device may comprise means for applying a disclosing substance, The means for applying the disclosing substance may be an injector incorporated into the device so that when a button is pressed, a disclosing substance, e.g. for the detection plaque, active caries or cancerous regions, is applied onto the surface of interest. The detection of light emitted from the surface of interest may occur simultaneously to the application of the disclosing substance. In one embodiment, the disclosing substance, e.g. for the detection of plaque, active caries or cancerous regions, may be released onto the surface through application of pressure, heat or contact with saliva. The disclosing substance may be a gel, a solution or a solid.

Preferably, the present invention is carried out in conditions of incident ambient light, wherein the filter and skirt features of the device assist in detection of the signal, shielding the device from the ambient light. The experiments detailed below demonstrate that the filter and skirt in combination improve the reduction of ambient light from the captured image and allow the luminescent and/or fluorescent source to be imaged in bright ambient light. The light sources used in the following experiments are blue Glowell® units (GLO-002 s/n 2090643 LUX Innovate) with an emission wavelength of 450 nm and light outputs of approximately 75 x 10⁶ (G1), 7.3 x 10⁶ (G2) and 0.6 x 10⁶ (G3) photons s⁻¹.

This shows that the light output of G2 is similar to the light output released from the disclosing solution applied to an extracted tooth with an active caries lesion.

### Experiment 1: Demonstration that the light from a G2 Glowell® is comparable to that of an active caries lesion from a recently extracted tooth.

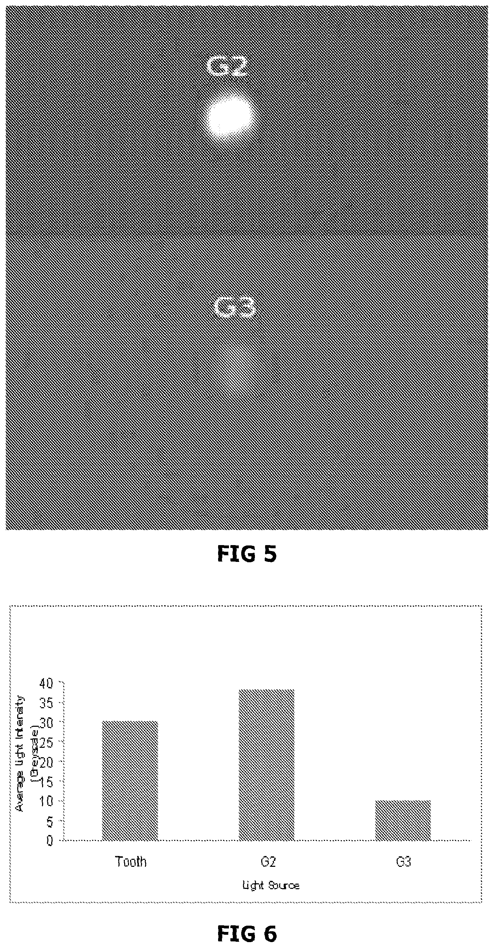

Figure 4 shows an extracted tooth that has been imaged with the CCD camera (SXVF-H9 Starlight Express Ltd) with an exposure time of 60 s and 2x2 binning after the addition of 250 µL of 1 mg cm⁻³ Aequorin in 1% Akucell Gel made up with 1 mM EDTA to the tooth surface. Figure 5 shows the G2 and G3 Glowell® units that have been imaged using the CCD camera with an exposure time of 60 s and 2x2 binning. The average light intensity (in greyscale units) was measured for a region of luminescence on the extracted tooth and for the G2 and G3 Glowell® units; this is shown in the graph in Figure 6. Figure 6 shows that the average light output values for the luminescent areas of the extracted tooth and the G2 and G3 Glowell® units were 30, 38 and 10 respectively. The light output of the G2 Glowell® is therefore comparable to that released from a tooth with active caries upon addition of the disclosing solution indicating that Glowell® units are a good model for testing.

### Experiment 2: The use of a skirt for imaging in ambient light.

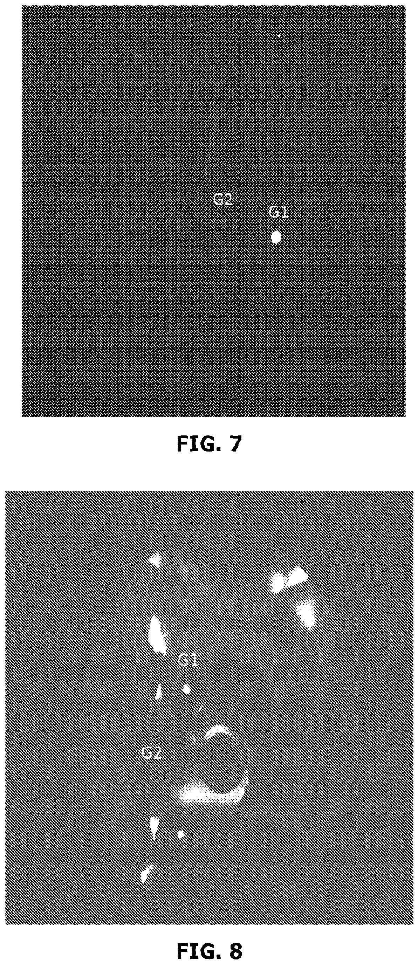

An endoscope (Olympus) was attached directly to the CCD camera by means of an endoscope - camera adapter (Pro Vision PVCC37 Camera Coupler Dart Systems). The endoscope has been modified using a prism so that the viewing angle is at 90 degrees rather than 0 degrees and so is in a format suitable for intraoral use. Three Glowell® units were placed directly in front of the endoscope input aperture. These were blue units with an emission wavelength of 450 nm and light outputs of approximately 75 x 10⁶ (G1), 7.3 x 10⁶ (C2) and 0.6x10⁶ (G3)photons s⁻¹. In order to capture images in near darkness (no ambient light) and so provide a control, the camera, endoscope and table supporting the Glowell® units were covered with a piece of black material. The experiment was carried out in a dark room, with all the lights switched off and the door closed. A 20 s exposure was taken with the camera set to 4x4 binning (Figure 7). The skirt was then attached to the distal end of the endoscope where the prism is housed and the orientation of the camera - endoscope altered to horizontal rather than vertical. A 20 s exposure was captured and 4x4 binning used with the lights switched on to represent ambient light (Figure 8). The light level at the endoscope was measured to be 450 lux using a light meter (Precision Gold), average ambient light from seven dental practioners was determined to be 400 lux. The skirt was then removed and a 20 s exposure with 4x4 binning was captured. The resulting image was saturated and no image could be resolved. Figures 7 and 8 demonstrate that the presence of the skirt allows images to be captured in ambient light using the camera - endoscope system, whereas without the skirt this is not possible.

### Experiment 3: The use of a filter with CCD camera and endoscope for imaging in ambient light.

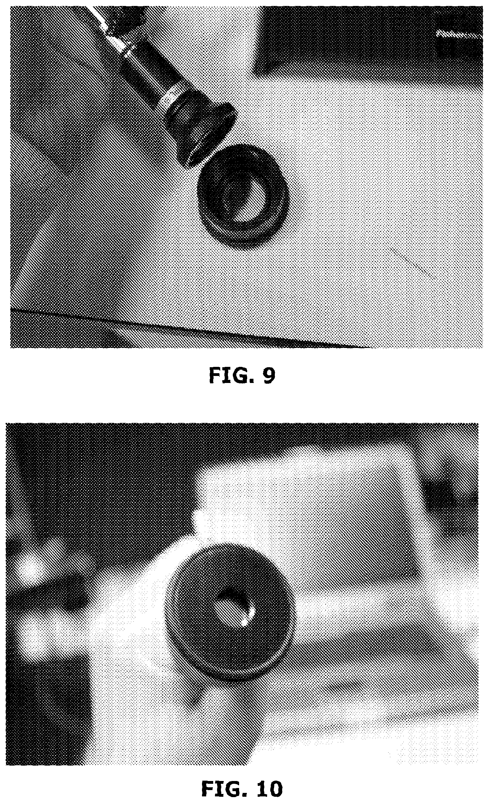

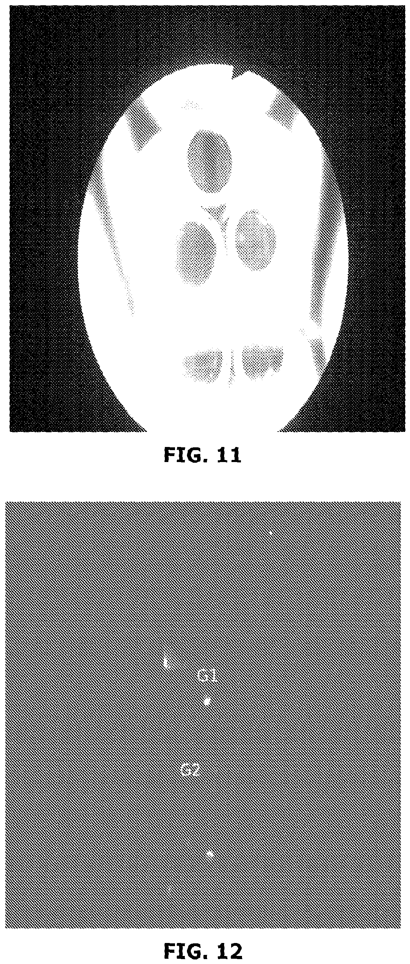

The same camera, endoscope and set up were used as for Experiment 2. An image was captured in ambient light (450 lux) without the skirt using a 5 s exposure time and 2x2 binning, A filter (Semrock Brightline series FF01-465/30) was inserted into the camera endoscope adapter as shown in Figures 9 and 10. The filter is a band pass filter and has a band width of 30 nm, centred on 465 nm. It therefore does not transmit light with a wavelength below 450 nm and above 480 nm. The positioning of the filter meant that all light reaching the camera CCD had passed through the filter. An image with the filter in place was captured using a 5 s exposure time and 2x2 binning (Figure 11). When no filter was used, the camera CCD became saturated and resolution of the image was lost. In Figure 11 there was some saturation but resolution of the image was possible. This demonstrates that the presence of the filter improves images captured in ambient light.

### Experiment 4: The use of the filter and skirt with the CCD camera and endoscope for imaging in ambient light.

The filter was left in place after experiment 3 and the skirt was reattached to the distal end of the endoscope. An image was captured with a 20 s exposure time with the binning set to 4x4 in ambient light (Figure 12). In Figure 12 light from the G1 and G2 Glowell® units are visible. In Figure 7 there was some interference from light entering from under the skirt (to the left of the brightest Glowell®); this problem was much reduced when the skirt and the filter were used in combination, see Figure 12. Since for intraoral use the fit may not always be completely tight then some stray ambient light may enter into the device and use of a filter helps to minimise any interference. Figure 11, indicates that a filter alone was not sufficient to reduce ambient light to a level at which the image is not saturated and so some resolution is lost. Figure 12 therefore shows that there is an advantage in using both the filter and skirt in combination when imaging in ambient light conditions. The filter alone is not enough to reduce ambient light and the filter and skirt in combination are better at reducing interference from ambient light than when the skirt alone is used.

### Experiment 5: The use of an imaging system comprising a hollow metal tube containing prism, lens, skirt and CCD.

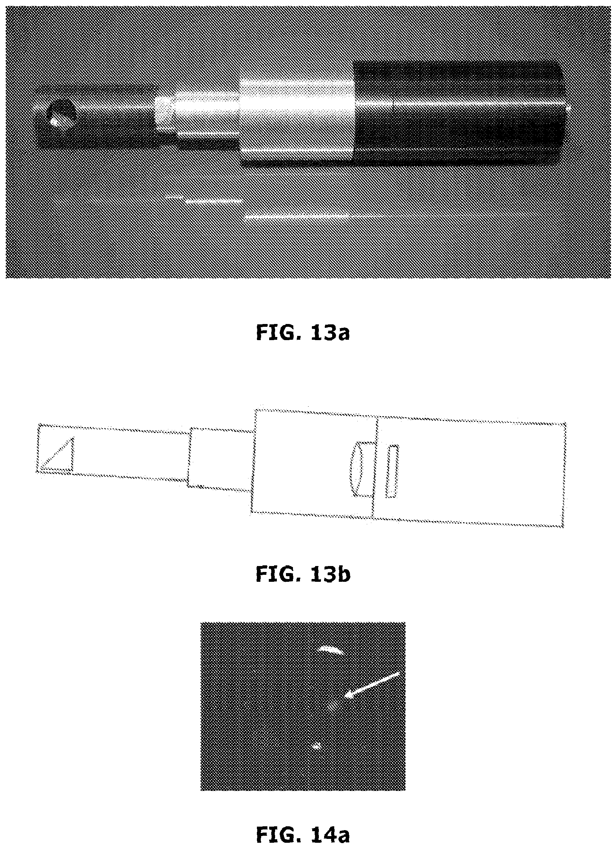

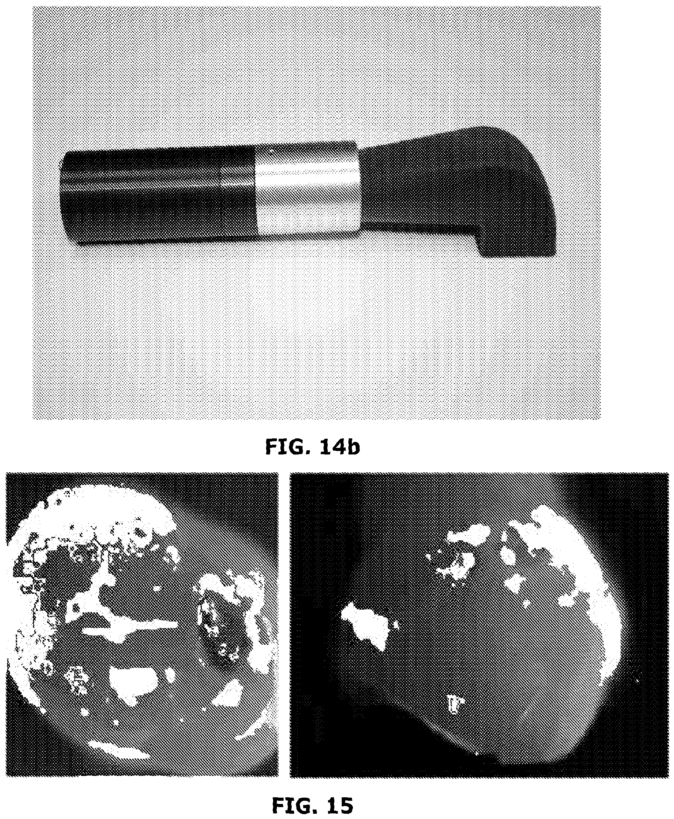

An image showing the luminescence emitted from the G2 Glowell® (position of light source shown with arrow) was captured in ambient light, using a skirt, see Figures 14a and 14b.

The imaging device shown in Figure 14b was used to capture a luminescence image (Figure 15) of caries activity on freshly extracted human teeth. 250 µL of Glowdent™ a formulation that luminescence in the presence of calcium ions, was placed onto the tooth surface. The luminescence images were obtained after 10 s, using 2x2 binning. After capture, the image was contrast enhanced using proprietary software. Distinct luminescence patterns (in white) were observed.

### Experiment 6: Investigating the influence of gel viscosity on the speed and magnitude of the light signal generated.

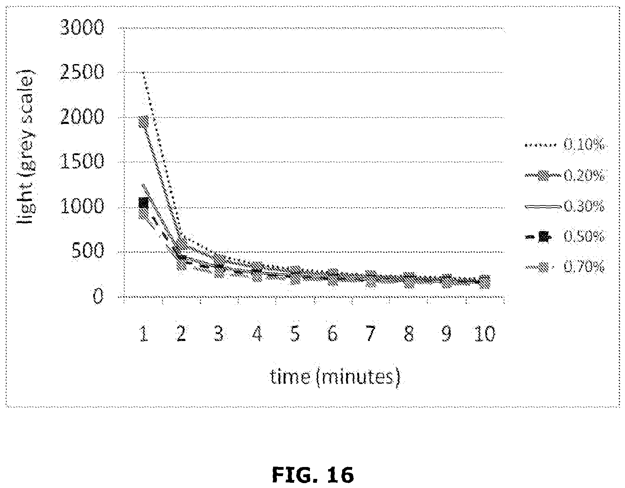

100 µL of Akucell® gel ranging in concentration from 0.3 to 0.7% and containing 10 µg/ml of protein was injected into the wells of a microwell plate into which 10 µL of 10 mM calcium acetate had been dried. A camera (SXVF-H9 Starlight Express Ltd) which had been placed in a dark box was used to capture images; 1 minute captures were made over a 10 minute period. Graphical representation of the results is shown in Figure 16 and clearly shows that the greater the viscosity of the gel the lower and slower the signal. Viscose gel can aid tooth imaging by ensuring the whole surface of a tooth is covered and therefore imaged and can prevent the gel quickly running down the tooth which may distort the image. It is clear, however, that greater signal is achieved with gels of lower viscosity ensuring that images from marker are captured, less viscose gels may also be more suitable to ensure the gel reaches the fissures of occlusal tooth surfaces.

### Experiment 7: investigating feasibility of shortening image capture times by merging successive images.

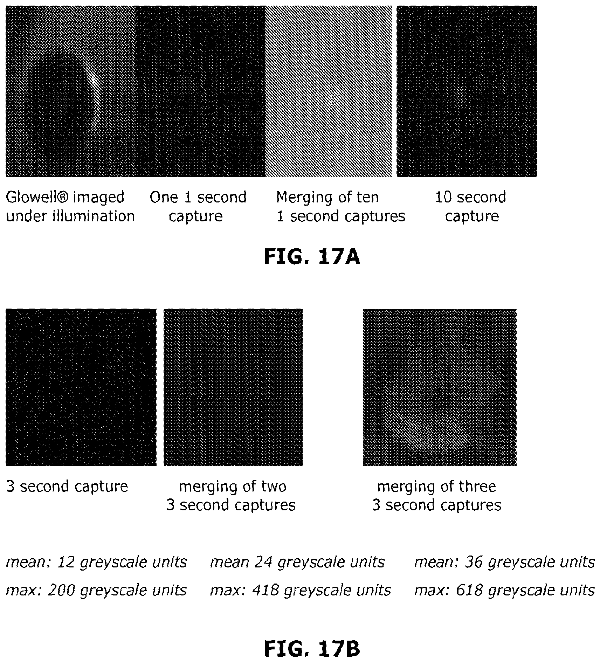

Images of a G1 Glowell (blue) were captured using the same device as outlined in Experiment 5. Images captured were: 3 s image under illuminated conditions; a single 1 second image; ten sequentially captured 1 second images; and a single 10 second capture, Figure 17A. The ten sequential images were merged by using the Arithmetic addition function in PaintShop Pro, no changes to contrast were made.

The occlusal surface of an extracted tooth was imaged after addition of 40 uL of Glowdent™, a formulation that luminesces in the presence of calcium ions. Three 3 second captures were obtained, using 2X2 binning. After capture, the images were merged (using proprietary imaging software), contrast enhanced (to the same level in all images), false coloured and data on light intensity (recorded as grayscale values) noted, Figure 17B. Distinct luminescence patterns (in white) were observed, with good resolution observed even on merged images.

The images in Figure 17 show that merging images captured over a shorter period can help improve light signal whilst minimising the potential for loss of image resolution from camera 'shake' over longer durations.

## Claims

1. A detection device comprising a proximal end (16) and a light-transmitting portion (18), wherein the proximal end (16) comprises a light detection means (6), the light-transmitting portion (18) comprising means for transmitting light along a path from a surface of a tooth to the light detection means (6), **characterised in that** the light detection means is adapted to detect a low light signal generated by luminescence in conditions of ambient light, wherein the device further comprises an injector or gun (12) configured to apply a disclosing substance comprising photoproteins to the surface of a tooth, wherein the injector or gun comprises a nozzle, wherein the light transmitting portion further comprises an LED light source configured to illuminate the surface of a tooth, wherein the device is configured to take an illuminated image prior to the detection and analysis of the low light signal generated by luminescence, wherein the LED light source has an off setting, wherein, in use, the device is configured to allow a user to turn off illumination by the LED light source to produce a luminescence image using the low light signal generated by luminescence, wherein the low light signal generated by luminescence is luminescent light emitted by the photoprotein in the disclosing substance that have come into contact with free ions that are released by the surface of a tooth due to demineralisation, wherein the low light signal generated by luminescence is detected in close succession to the application of the disclosing substance to the surface of a tooth, wherein the device is configured to compare the luminescence image with the illuminated image in order to determine the location of the demineralisation on the surface of a tooth.

2. A device according to claim 1, wherein the light transmitting portion (18) is positioned adjacent to and is adapted to fit around the surface of a tooth.

3. A device according to any one of the preceding claims, wherein the proximal end (16) further comprises a lens (3), wherein the lens (3) is located at a position along the path for transmitting light from a surface of a tooth to the light detection means (6).

4. A device according to any one of the preceding claims, wherein the means for transmitting light is a prism or reflective surface (2).

5. A device according to claim 4, wherein the prism or reflective surface (2) is coated with a bandpass, polarising or dichroic filter material.

6. A device according to claim 5, wherein the prism or reflective surface (2) is positioned upstream of the light detection means (6).

7. A device according to any one of the preceding claims, wherein the light-transmitting portion (18) is arranged at an angle permitting light from a specific area of the surface of a tooth to be received.

8. A device according to any one of the preceding claims, wherein the device further comprises a skirt 10 surrounding an end of the light-transmitting portion (18).

9. A device according to any one of the preceding claims, wherein the light detection means (6) is sensitive to a lower detection limit of a rate of 9.63 x 10⁶ photons s⁻¹.

10. A device according to any one of the preceding claims, wherein the light detection means (6) is selected from a group consisting of a luminometer, fluorometer, spectrophotometer, camera, charge coupled device (CCD), complementary metal-oxide semiconductor (CMOS), charged injection device (CID), avalanche photodiode (APD), single photon detector, digital camera, intensified camera, photographic film, photometric detector, photomultiplier tubes (PMT), microelectromechanical system (MEMS), a human eye or a combination of these.

11. A device according to any one of the preceding claims, wherein the light-transmitting portion (18) comprises at least one light source selected from the group consisting of an LED, laser, lamp or a hole cut into the device to allow ambient light to enter therein, thereby allowing transmittance of light onto the surface to be assessed.

12. A device according to any one of the preceding claims, wherein the device further comprises a camera.

13. A kit comprising a detection device in accordance with any one of the preceding claims.

14. A method of detecting a low light signal generated by luminescence from a disclosing substance comprising a photoprotein which has been applied to a surface of a tooth, wherein the disclosing substance produces a luminescent signal in the presence of free ions, wherein the detection is performed using a detection device as claimed in any of claims 1 to 12, the method comprising the steps of:
a. placing the light-transmitting portion (18) of the device adjacent to the tooth surface;
b. taking an image using an LED light source and then turning off the LED light source;
c. applying the disclosing substance to the surface of a tooth, wherein the disclosing substance is released from the detection device and applied to the tooth surface as a fine spray using an injector or gun (12) from the device comprising a nozzle;
d. detecting the luminescence emitted from the tooth surface; and
e. determining the location of demineralisation on the tooth surface.

15. A method according to claim 14, further comprising the step of producing an image of the surface of a tooth by focussing the light emitted therefrom through a lens (3) provided on the device.

16. A method according to claim, 14 or 15, further comprising the step of producing an image of the surface of a tooth prior to application of the disclosing substance.

17. A method according to any one of claims 14 to 16, wherein the disclosing substance comprises a powder that dissolves in the presence of saliva.

18. A method according to claims 14 to 17, wherein the low light signal is detected in close succession to the application of the disclosing substance to the surface of a tooth.

## Patentansprüche

1. Detektorvorrichtung mit einem proximalen Ende (16) und einem lichtaussendenden Abschnitt (18), wobei das proximale Ende (16) ein Lichtdetektormittel (6) und der lichtaussendende Abschnitt (18) Mittel zum Aussenden von Licht entlang eines Wegs von einer Oberfläche eines Zahns zu dem Lichtdetektormittel (6) umfasst, **dadurch gekennzeichnet, dass** das Lichtdetektormittel so ausgelegt ist, dass es ein Schwachlichtsignal erkennt, das unter Umgebungslichtbedingungen durch Lumineszenz generiert wird, wobei die Vorrichtung ferner einen Injektor oder eine Pistole (12) umfasst, der/die so konfiguriert ist, dass er/sie eine Photoproteine umfassende Markierungssubstanz auf die Oberfläche eines Zahns aufbringt, wobei der Injektor oder die Pistole eine Düse umfasst, wobei der lichtaussendende Abschnitt ferner eine LED-Lichtquelle umfasst, die so konfiguriert ist, dass sie die Oberfläche eines Zahns beleuchtet, wobei die Vorrichtung so konfiguriert ist, dass sie vor der Erkennung und Analyse des durch Lumineszenz generierten Schwachlichtsignals ein beleuchtetes Bild aufnimmt, wobei die LED-Lichtquelle eine Aus-Stellung aufweist, wobei die Vorrichtung so konfiguriert ist, dass sie es einem Benutzer ermöglicht, im Gebrauch die Beleuchtung durch die LED-Lichtquelle auszuschalten und so unter Verwendung des durch die Lumineszenz generierten Schwachlichtsignals ein Lumineszenzbild zu erzeugen, wobei es sich bei dem durch die Lumineszenz generierten Schwachlichtsignal um Lumineszenzlicht handelt, das von den Photoproteinen in der Markierungssubstanz emittiert wird, die mit freien Ionen in Kontakt gekommen sind, welche aufgrund von Entmineralisierung von einer Oberfläche eines Zahns abgegeben werden, wobei das durch Lumineszenz generierte Schwachlichtsignal unmittelbar nach dem Aufbringen der Markierungssubstanz auf die Oberfläche eines Zahns erkannt wird, wobei die Vorrichtung so konfiguriert ist, dass sie das Lumineszenzbild mit dem beleuchteten Bild vergleicht, um die Lage der Entmineralisierung an der Oberfläche eines Zahns zu ermitteln.

2. Vorrichtung nach Anspruch 1, wobei der lichtaussendende Abschnitt (18) an die Oberfläche eines Zahns angelegt wird und so ausgelegt ist, dass er um diese herumpasst.

3. Vorrichtung nach einem der vorhergehenden Ansprüche, wobei das proximale Ende (16) ferner eine Linse (3) umfasst, wobei die Linse (3) in einer Position entlang des Wegs zum Aussenden von Licht von einer Oberfläche eines Zahns zu dem Lichtdetektormittel (6) angeordnet ist.

4. Vorrichtung nach einem der vorhergehenden Ansprüche, wobei es sich bei dem Mittel zum Aussenden von Licht um ein Prisma oder eine reflektierende Oberfläche (2) handelt.

5. Vorrichtung nach Anspruch 4, wobei das Prisma oder die reflektierende Oberfläche (2) mit einem Bandpass-, Polarisations- oder dichroitischen Filtermaterial beschichtet ist.

6. Vorrichtung nach Anspruch 5, wobei das Prisma oder die reflektierende Oberfläche (2) vor dem Lichtdetektormittel (6) positioniert ist.

7. Vorrichtung nach einem der vorhergehenden Ansprüche, wobei der lichtaussendende Abschnitt (18) in einem Winkel angeordnet ist, der das Empfangen von Licht aus einem bestimmten Bereich an der Oberfläche eines Zahns ermöglicht.

8. Vorrichtung nach einem der vorhergehenden Ansprüche, wobei die Vorrichtung ferner eine Schürze (10) umfasst, die ein Ende des lichtaussendenden Abschnitts (18) umgibt.

9. Vorrichtung nach einem der vorhergehenden Ansprüche, wobei das Lichtdetektormittel (6) bis zu einem unteren Detektionsratengrenzwert von 9,63 x 10⁶ Photonen s⁻¹ empfindlich ist.

10. Vorrichtung nach einem der vorhergehenden Ansprüche, wobei das Lichtdetektormittel (6) aus einer Gruppe ausgewählt ist, die aus einem Luminometer, einem Fluorometer, einem Spektralphotometer, einer Kamera, einem ladungsgekoppelten Bauelement (CCD), einem komplementären Metall-Oxid-Halbleiter (CMOS), einem Charge Injection Device (CID), einer Avalanche-Photodiode (APD), einem Einzelphotonendetektor, einer Digitalkamera, einer verstärkten Kamera, einem fotografischen Film, einem photometrischen Detektor, Photoelektronenvervielfachem (PMT), einem Mikrosystem (MEMS), einem menschlichen Auge oder einer Kombination davon besteht.

11. Vorrichtung nach einem der vorhergehenden Ansprüche, wobei der lichtaussendende Abschnitt (18) mindestens eine Lichtquelle umfasst, die aus der Gruppe ausgewählt ist, welche aus einer LED, einem Laser, einer Lampe oder einer in die Vorrichtung hineingeschnittenen Öffnung besteht, durch die Umgebungslicht eindringen kann, wodurch Licht auf die zu überprüfende Oberfläche ausgesendet wird.

12. Vorrichtung nach einem der vorhergehenden Ansprüche, wobei die Vorrichtung ferner eine Kamera umfasst.

13. Ausrüstung, die eine Detektorvorrichtung nach einem der vorhergehenden Ansprüche umfasst.

14. Verfahren zum Erkennen eines Schwachlichtsignals, das durch Lumineszenz von einer Markierungssubstanz generiert wird, die ein Photoprotein umfasst, die auf eine Oberfläche eines Zahns aufgebracht wurde, wobei die Markierungssubstanz in Anwesenheit von freien Ionen ein Lumineszenzsignal erzeugt, wobei das Erkennen unter Verwendung einer Detektorvorrichtung nach einem der Ansprüche 1 bis 12 erfolgt, wobei das Verfahren folgende Schritte umfasst:
a) Anlegen des lichtaussendenden Abschnitts (18) der Vorrichtung an die Zahnoberfläche,
b) Aufnehmen eines Bildes unter Verwendung einer LED-Lichtquelle und danach Ausschalten der LED-Lichtquelle,
c) Aufbringen der Markierungssubstanz auf die Oberfläche eines Zahns, wobei die Markierungssubstanz von der Detektorvorrichtung abgegeben und unter Verwendung eines Injektors oder einer Pistole (12) von der Vorrichtung mit einer Düse als feines Spray auf die Zahnoberfläche aufgebracht wird,
d) Erkennen der von der Zahnoberfläche emittierten Lumineszenz und
e) Ermitteln der Lage einer Entmineralisierung an der Zahnoberfläche.

15. Verfahren nach Anspruch 14, das ferner das Erzeugen eines Bildes von der Oberfläche eines Zahns durch Fokussieren des davon emittierten Lichts durch eine an der Vorrichtung vorgesehene Linse (3) umfasst.

16. Verfahren nach Anspruch 14 oder 15, das ferner das Erzeugen eines Bildes von der Oberfläche eines Zahns vor dem Aufbringen der Markierungssubstanz umfasst.

17. Verfahren nach einem der Ansprüche 14 bis 16, wobei die Markierungssubstanz ein Pulver umfasst, das sich in Anwesenheit von Speichel auflöst.

18. Verfahren nach den Ansprüchen 14 bis 17, wobei das Schwachlichtsignal unmittelbar nach dem Aufbringen der Markierungssubstanz auf die Oberfläche eines Zahns erkannt wird.

## Revendications

1. Dispositif de détection comprenant une extrémité proximale (16) et une partie de transmission de lumière (18), dans lequel l'extrémité proximale (16) comprend un moyen de détection de lumière (6), la partie de transmission de lumière (18) comprenant des moyens pour transmettre de la lumière le long d'un trajet à partir d'une surface d'une dent vers le moyen de détection de lumière (6), **caractérisé en ce que** le moyen de détection de lumière est adapté à détecter un signal à faible lumière généré par luminescence dans des conditions de lumière ambiante, dans lequel le dispositif comprend en outre un injecteur ou pistolet (12) configuré pour appliquer une substance révélatrice comprenant des photoprotéines sur la surface d'une dent, dans lequel l'injecteur ou pistolet comprend une buse, dans lequel la partie de transmission de lumière comprend en outre une source de lumière à DEL configurée pour éclairer la surface d'une dent, dans lequel le dispositif est configuré pour prendre une image éclairée avant la détection et l'analyse du signal à faible lumière généré par luminescence, dans lequel la source de lumière à DEL comprend une fonction de mise hors tension, dans lequel, lors de l'utilisation, le dispositif est configuré pour permettre à un utilisateur d'éteindre l'éclairage par la source de lumière à DEL pour produire une image de luminescence au moyen du signal à faible lumière généré par luminescence, dans lequel le signal à faible lumière généré par luminescence est une lumière luminescente émise par la photoprotéine dans la substance révélatrice qui est entrée en contact avec des ions libres qui sont libérés par la surface d'une dent en raison d'une déminéralisation, dans lequel le signal à faible lumière généré par luminescence est détecté en succession rapprochée à l'application de la substance révélatrice sur la surface d'une dent, dans lequel le dispositif est configuré pour comparer l'image de luminescence à l'image éclairée afin de déterminer l'emplacement de la déminéralisation sur la surface d'une dent.

2. Dispositif selon la revendication 1, dans lequel la partie de transmission de lumière (18) est positionnée adjacente à et est adaptée à s'ajuster autour de la surface d'une dent.

3. Dispositif selon l'une quelconque des revendications précédentes, dans lequel l'extrémité proximale (16) comprend en outre une lentille (3), dans lequel la lentille (3) est située au niveau d'une position le long du trajet pour transmettre une lumière à partir d'une surface d'une dent au moyen de détection de lumière (6).

4. Dispositif selon l'une quelconque des revendications précédentes, dans lequel les moyens pour transmettre de la lumière sont un prisme ou une surface réfléchissante (2).

5. Dispositif selon la revendication 4, dans lequel le prisme ou la surface réfléchissante (2) est revêtu(e) d'un matériau filtrant passe-bande, polarisant ou dichroïque.

6. Dispositif selon la revendication 5, dans lequel le prisme ou la surface réfléchissante (2) est positionné(e) en amont du moyen de détection de lumière (6).

7. Dispositif selon l'une quelconque des revendications précédentes, dans lequel la partie de transmission de lumière (18) est agencée selon un angle permettant la réception de lumière à partir d'une zone spécifique de la surface d'une dent.

8. Dispositif selon l'une quelconque des revendications précédentes, dans lequel le dispositif comprend en outre une jupe (10) entourant une extrémité de la partie de transmission de lumière (18).

9. Dispositif selon l'une quelconque des revendications précédentes, dans lequel le moyen de détection de lumière (6) est sensible à une limite de détection inférieure d'une vitesse de 9,63 x 10⁶ photons s⁻¹.

10. Dispositif selon l'une quelconque des revendications précédentes, dans lequel le moyen de détection de lumière (6) est sélectionné parmi un groupe composé d'un luminomètre, d'un fluoromètre, d'un spectrophotomètre, d'une caméra, d'un dispositif à couplage de charge (CCD), d'un semiconducteur complémentaire à l'oxyde de métal (CMOS), d'un dispositif d'injection de charge (CID), d'une photodiode à avalanche (APD), d'un détecteur monophotonique, d'une caméra numérique, d'une caméra intensifiée, d'un film photographique, d'un détecteur photométrique, de tubes photomultiplicateurs (PMT), d'un système microélectromécanique (MEMS), d'un oeil humain ou d'une combinaison de ceux-ci.

11. Dispositif selon l'une quelconque des revendications précédentes, dans lequel la partie de transmission de lumière (18) comprend au moins une source de lumière sélectionnée parmi le groupe composé d'une DEL, d'un laser, d'une lampe ou d'un trou découpé dans le dispositif pour permettre l'entrée de lumière ambiante à l'intérieur, ce qui permet d'évaluer la transmittance de lumière sur la surface.

12. Dispositif selon l'une quelconque des revendications précédentes, dans lequel le dispositif comprend en outre une caméra.

13. Kit comprenant un dispositif de détection selon l'une quelconque des revendications précédentes.

14. Procédé de détection d'un signal à faible lumière généré par luminescence à partir d'une substance révélatrice comprenant une photoprotéine qui a été appliquée sur une surface d'une dent, dans lequel la substance révélatrice produit un signal luminescent en présence d'ions libres, dans lequel la détection est réalisée au moyen d'un dispositif de détection selon l'une quelconque des revendications 1 à 12, le procédé comprenant les étapes de :
a. placement de la partie de transmission de lumière (18) du dispositif de manière adjacente à la surface de dent ;
b. prise d'une image au moyen d'une source de lumière à DEL puis extinction de la source de lumière à DEL ;
c. application de la substance révélatrice sur la surface d'une dent, dans lequel la substance révélatrice est libérée du dispositif de détection et appliquée sur la surface de dent sous la forme d'une pulvérisation fine au moyen d'un injecteur ou pistolet (12) à partir du dispositif comprenant une buse ;
d. détection de la luminescence émise à partir de la surface de dent ; et
e. détermination de l'emplacement de déminéralisation sur la surface de dent.

15. Procédé selon la revendication 14, comprenant en outre l'étape de production d'une image de la surface d'une dent en dirigeant la lumière émise à partir de celle-ci à travers une lentille (3) disposée sur le dispositif.

16. Procédé selon la revendication 14 ou 15, comprenant en outre l'étape de production d'une image de la surface d'une dent avant l'application de la substance révélatrice.

17. Procédé selon l'une quelconque des revendications 14 à 16, dans lequel la substance révélatrice comprend une poudre qui se dissout en présence de salive.

18. Procédé selon les revendications 14 à 17, dans lequel le signal à faible lumière est détecté en succession rapprochée à l'application de la substance révélatrice sur la surface d'une dent.
